# EUROPEAN PATENT APPLICATION

(11) **EP 4 417 696 A1**
(43) Date of publication of application: **21.08.2024**
(21) Application number: 22881178.2
(22) Date of filing: 29.07.2022
(51) Int. Cl.: C12N 15/77, C12N 9/88, C12P 13/08

(54) **CORYNEBACTERIUM GLUTAMICUM VARIANT HAVING IMPROVED L-LYSINE PRODUCTION ABILITY AND METHOD FOR PRODUCING L-LYSINE BY USING SAME**

(30) Priority: 14.10.2021 KR 20210136768
(71) Applicant: Daesang Corporation, Seoul 02586 (KR)
(72) Inventor: RYU, Mi, Icheon-si, Gyeonggi-do 17384 (KR); HONG, In Pyo, Icheon-si, Gyeonggi-do 17384 (KR); KIM, Bong Ki, Seoul 07900 (KR); PARK, Seok Hyun, Namyangju-si, Gyeonggi-do 12258 (KR); PARK, Joon Hyun, Seongnam-si, Gyeonggi-do 13523 (KR)
(74) Representative: Cabinet Nony
(86) International application number: PCT/KR2022/011177
(87) International publication number: WO 2023/063547

(57) **Abstract**

The present invention relates to a *Corynebacterium glutamicum* mutant strain having enhanced L-lysine productivity and a method of producing L-lysine using the same. The *Corynebacterium glutamicum* mutant strain is able to produce L-lysine in an improved yield as a result of increasing the supply of the L-lysine precursor and sugar utilization by increasing or enhancing the expression of the gene encoding enolase and/or reducing or weakening the expression of the gluconate operon transcriptional repressor.

## Description

### Technical Field

The present invention relates to a *Corynebacterium glutamicum* mutant strain having enhanced L-lysine productivity and a method of producing L-lysine using the same.

### Background Art

L-lysine is an essential amino acid that is not synthesized in the human or animal body. L-lysine needs to be supplied externally and is generally produced by fermentation using microorganisms such as bacteria or yeast. L-lysine production may be performed using naturally occurring wild-type strains or mutant strains obtained by modifying the wild-type strains to have enhanced L-lysine productivity. In recent years, in order to improve the production efficiency of L-lysine, various recombinant strains or mutant strains having excellent L-lysine productivity and methods of producing L-lysine using the recombinant strains or mutant strains have been developed by applying gene recombination technology to microorganisms such as *Escherichia coli* and *Corynebacterium,* which are widely used for the production of L-amino acids and other useful substances.

According to Korean Patent Nos. 10-0838038 and 10-2139806, L-lysine productivity may be enhanced by increasing the expression of genes encoding proteins, including L-lysine production-related enzymes, through modification of the nucleotide sequences of the genes or the amino acid sequences of the proteins, or by removing unnecessary genes. In addition, Korean Patent Application Publication No. 10-2020-0026881 discloses a method by which the existing promoter of a gene encoding an enzyme involved in L-lysine production is changed to a promoter having strong activity in order to increase the expression of the gene.

As described above, various methods for increasing L-lysine productivity have been developed, but there are dozens of types of proteins such as enzymes, transcription factors, and transport proteins, which are directly or indirectly involved in L-lysine production. Hence, extensive studies still need to be conducted on whether or not changes in the activities of these proteins lead to an increase in L-lysine productivity.

### [Prior Art Documents]

### [Patent Documents]

Korean Patent No. 10-0838038
Korean Patent No. 10-2139806
Korean Patent Application Publication No. 10-2020-0026881

### DISCLOSURE

### Technical Problem

An object of the present disclosure is to provide a *Corynebacterium glutamicum* mutant strain having enhanced L-lysine productivity.

Another object of the present disclosure is to provide a method of producing L-lysine using the mutant strain.

### Technical Solution

The present inventors have conducted studies to develop a novel mutant strain having enhanced L-lysine productivity using a *Corynebacterium glutamicum* strain, and as a result, have found that, when the gene sequence of the Eno gene encoding enolase involved in the L-lysine biosynthesis pathway, especially the start codon, is replaced, the production of L-lysine increases, and additionally, an amino acid at a specific position in the amino acid sequence of the gntR gene encoding the gluconate operon transcriptional repressor is substituted, the production of L-lysine further increases, thereby completing the present disclosure.

One aspect of the present disclosure provides a *Corynebacterium glutamicum* mutant strain having enhanced L-lysine productivity by having enhanced activity of enolase.

"Enolase" as used in the present invention is also called phosphopyruvate hydratase, and refers to an enzyme that catalyzes a reaction that converts 2-phosphoglycerate (2-PG) to phosphoenolpyruvate (PEP) during glycolysis to supply pyruvate to the TCA cycle.

According to one embodiment of the present disclosure, the enolase may be derived from a *Corynebacterium* sp. strain. Specifically, the *Corynebacterium* sp. strain may be, but is not limited to, *Corynebacterium glutamicum, Corynebacterium crudilactis, Corynebacterium deserti, Corynebacterium callunae, Corynebacterium suranareeae, Corynebacterium lubricant is, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens.*

"Enhanced activity" as used in the present invention means that the expression of a gene of interest encoding a protein such as an enzyme, transcription factor or transport protein is newly introduced or increased, so that the expression level of the gene is increased compared to that in the wild-type strain or the strain before modification. This enhanced activity also includes: a case in which the activity of the protein itself is increased compared to the activity of the protein of the parent microorganism by substitution, insertion, deletion, or a combination thereof of one or more nucleotides in the gene encoding the protein; a case in which the overall enzyme activity in the cell is higher than that in the wild-type strain or the strain before modification due to increased expression or translation of the gene encoding the protein; and a combination thereof.

According to one embodiment of the present invention, the enhanced activity of the enolase may be achieved by site-directed mutagenesis of the gene encoding the enolase.

According to one embodiment of the present invention, the gene encoding the enolase may be represented by the nucleotide sequence of SEQ ID NO: 1.

According to one embodiment of the present invention, the gene encoding the enolase may be represented by the amino acid sequence of SEQ ID NO: 2.

According to one embodiment of the present invention, the enhanced activity of the enolase may be achieved by substitution of one or more nucleotides among 1 to 100 nucleotides in the nucleotide sequence of the gene encoding the enolase.

More specifically, the genetic mutation in the present disclosure may be achieved by substitution of 1 to 100, preferably 1 to 10 consecutive or non-consecutive nucleotides in the nucleotide sequence of the gene encoding the enolase.

According to one embodiment of the present invention, the enhanced activity of the enolase may be achieved by replacing the start codon of the gene encoding enolase with ATG.

In one example of the present invention, a *Corynebacterium glutamicum* mutant strain having a new start codon of the Eno gene was obtained by GTG-to-ATG replacement in the start codon in the nucleotide sequence of SEQ ID NO: 1, which is the nucleotide sequence of the Eno gene encoding enolase of a *Corynebacterium glutamicum* strain. This *Corynebacterium glutamicum* mutant strain may contain an enolase gene having the nucleotide sequence of SEQ ID NO: 3 or encoding the amino acid sequence of SEQ ID NO: 4.

This *Corynebacterium glutamicum* mutant strain having the mutation in the enolase gene may have enhanced L-lysine productivity.

As used herein, "enhanced productivity" means that L-lysine productivity of the mutant strain is higher than that of the parent strain. The parent strain refers to a wild-type strain to be mutated or a mutant strain, and includes a strain that is to be mutated directly or to be transformed with a recombinant vector or the like. In the present invention, the parent strain may be a wild-type *Corynebacterium glutamicum* strain or a strain mutated from the wild-type strain.

According to one embodiment of the present invention, the parent strain may be a mutant strain having mutations in the sequences of genes (e.g., lysC, zwf and horn genes) that are involved in lysine production. Specifically, the parent strain may be a *Corynebacterium glutamicum* strain (hereinafter referred to as "*Corynebacterium glutamicum* DS1 strain") deposited with the Korean Culture Center of Microorganisms on April 2, 2021 under accession number KCCM12969P.

In one example of the present disclosure, as the *Corynebacterium glutamicum* mutant strain having enhanced L-lysine productivity contains a start codon mutation in the enolase (Eno) gene, it may exhibit increased L-lysine productivity compared to the parent strain due to an increase in the supply of PEP, an important precursor of L-lysine. In particular, the *Corynebacterium glutamicum* mutant strain may show an increase in L-lysine production of 5% or more, specifically 5 to 400 (preferably 10 to 30%), compared to the parent strain, and thus produce 55 to 85 g, preferably 60 to 80 g of L-lysine, per liter of the strain culture medium.

In addition, in the present invention, mutations may be induced in other genes involved in L-lysine biosynthesis in addition to the Eno gene encoding enolase in order to further improve the L-lysine productivity.

According to one embodiment of the present invention, the mutant strain may further have weakened activity of the gluconate operon transcriptional repressor.

In microorganisms, protein expression is regulated by transcription of an operon composed of regulatory genes, operators, promoters, structural genes, etc. When transcriptional activation is determined, a series of proteins that induce the activation of inactivated genes and facilitate smooth transcription are called transcriptional activators, and conversely, a series of proteins that are produced to deactivate activated genes are called transcriptional repressors. The "gluconate operon transcriptional repressor" used in the present invention is a regulatory protein involved in gluconate metabolism, sugar influx, etc., and serves to inhibit mRNA synthesis by binding to the operator of the gluconate operon.

According to one embodiment of the present invention, the gluconate operon transcriptional repressor may be derived from a *Corynebacterium* sp. strain. Specifically, the *Corynebacterium* sp. strain may be, but is not limited to, *Corynebacterium glutamicum, Corynebacterium crudilactis, Corynebacterium deserti, Corynebacterium callunae, Corynebacterium suranareeae, Corynebacterium lubricant is, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens.*

"Weakened activity" as used in the present invention means that the expression of a gene of interest encoding a protein such as an enzyme, transcription factor or transport protein is inhibited or removed, so that the expression level of the gene is decreased compared to that in the wild-type strain or the strain before modification. This weakened activity also includes: a case in which the activity of the protein itself is decreased compared to the activity of the protein of the parent microorganism by substitution, insertion, deletion, or a combination thereof of one or more nucleotides in the gene encoding the protein; a case in which the overall enzyme activity in the cell is lower than that in the wild-type strain or the strain before modification due to inhibited expression or translation of the gene encoding the protein; and a combination thereof.

According to one embodiment of the present invention, the weakened activity of the gluconate operon transcriptional repressor may be achieved by site-directed mutagenesis of the gene encoding the gluconate operon transcriptional repressor.

According to one embodiment of the present invention, the gene encoding the gluconate operon transcriptional repressor may be represented by the nucleotide sequence of SEQ ID NO: 5.

According to one embodiment of the present invention, the gene encoding the gluconate operon transcriptional repressor may be represented by the amino acid sequence of SEQ ID NO: 6.

According to one embodiment of the present invention, the weakened activity of the gluconate operon transcriptional repressor may be achieved by substitution of one or more amino acids in the 10^{th} to 100^{th} amino acid region in the amino acid sequence of the gene encoding the gluconate operon transcriptional repressor.

More specifically, the genetic mutation in the present invention may be achieved by substitution of one or more amino acids in the amino acid sequence of the gene encoding the gluconate operon transcriptional repressor, preferably substitution of 1, 2, 3, 4, or 5 consecutive or non-consecutive amino acids in the 10^{th} to 100^{th}, 20^{th} to 90^{th}, or 30^{th} to 80^{th} amino acid region in the amino acid sequence.

In one example of the present disclosure, a *Corynebacterium glutamicum* mutant strain having a new amino acid sequence of the gntR gene was obtained by serine (Ser)-to-phenylalanine (Phe) substitution at position 77 in the amino acid sequence of the gntR gene encoding the gluconate operon transcriptional repressor of a *Corynebacterium glutamicum* strain. This *Corynebacterium glutamicum* mutant strain may contain a gluconate operon transcriptional repressor gntR gene having the nucleotide sequence of SEQ ID NO: 7 or encoding the amino acid sequence of SEQ ID NO: 8.

Here, the parent strain may be a *Corynebacterium glutamicum* mutant strain having a mutation in the sequence of the Eno gene encoding enolase.

In one example of the present invention, as the *Corynebacterium glutamicum* mutant strain having enhanced L-lysine productivity contains an amino acid mutation in the gluconate operon transcriptional repressor gntR gene together with a start codon mutation in the enolase (Eno) gene, it may have exhibit increased L-lysine productivity compared to the parent strain containing only a start codon mutation in the Eno gene encoding enolase due to increased sugar utilization of the strain. In particular, the *Corynebacterium glutamicum* mutant strain may show an increase in L-lysine production of 2% or more, specifically 2 to 20%, compared to the parent strain, and thus produce 63 to 85 g, preferably 65 to 80 g of L-lysine, per liter of the strain culture medium.

The *Corynebacterium glutamicum* mutant strain according to one embodiment of the present invention may be obtained through a recombinant vector containing a variant resulting from replacement of the start codon of the enolase (Eno) gene in the parent strain and/or a variant resulting from substitution of a portion of the amino acid sequence of the gluconate operon transcriptional repressor gntR gene in the parent strain.

"Portion" as used in the present invention means not all of the amino acid sequence, nucleotide sequence, or polynucleotide sequence, and may be, but is not limited to, 1 to 300, preferably 1 to 100, more preferably 1 to 50.

"Variant" as used in the present invention refers to a variant resulting from replacement of the start codon GTG of the enolase (Eno) gene, which is involved in L-lysine biosynthesis, with ATG, and/or substitution of one or more amino acids in the 10^{th} to 100^{th} amino acid region in the amino acid sequence of the gluconate operon transcriptional repressor gntR gene.

According to one embodiment of the present invention, the variant resulting from replacement of the start codon of the enolase gene with ATG may have the nucleotide sequence of SEQ ID NO: 3 or the amino acid sequence of SEQ ID NO: 4.

According to one embodiment of the present invention, the variant resulting from substitution of the amino acid at position 77 in the amino acid sequence of the gluconate operon transcriptional repressor gene may have the nucleotide sequence of SEQ ID NO: 7 or the amino acid sequence of SEQ ID NO: 8.

"Vector" as used in the present invention refers to an expression vector capable of expressing a protein of interest in a suitable host cell, and means a gene construct that contains essential regulatory elements operably linked so that an inserted gene is expressed. Here, "operably linked" means that a gene to be expressed and the regulatory sequence thereof are functionally linked to each other in a manner enabling gene expression. "Regulatory elements" includes a promoter for initiating transcription, any operator sequence for controlling transcription, a sequence encoding suitable mRNA ribosome binding sites, and a sequence for controlling termination of transcription and translation. Examples of this vector include, but are not limited to, plasmid vectors, cosmid vectors, bacteriophage vectors, and viral vectors.

"Recombinant vector" as used in the present invention refers to a recombinant vector that may be transformed into a suitable host cell, and then may replicate regardless of the genome of the host cell or may be integrated into the genome itself. In this case, the "suitable host cell" may contain a replication origin, which is a particular nucleotide sequence which enables the vector to replicate in the suitable host cell and from which replication starts.

The transformation may be performed using a suitable vector introduction technique selected depending on the host cell, so that the gene of interest may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or a combination thereof. For the transformed gene, there is no limitation on whether the gene is inserted into the chromosome of the host cell or located outside of the chromosome, as long as the gene may be expressed in the host cell.

The host cell may include a cell transfected, transformed, or infected with the recombinant vector or polynucleotide of the present invention *in vivo* or *in vitro.* The host cell containing the recombinant vector of the present invention may be a recombinant host cell, a recombinant cell, or a recombinant microorganism.

In addition, the recombinant vector according to the present invention may contain a selection marker. The selection marker may be used to select a transformant (host cell) obtained by transformation with the vector. Since only cells expressing the selection marker may survive in the medium treated with the selection marker, the selection marker may select the transformed cells. Representative examples of the selection marker include, but are not limited to, kanamycin, streptomycin, and chloramphenicol.

Genes inserted into the recombinant vector for transformation according to the present invention may be substituted into a host cell such as a *Corynebacterium* sp. microorganism by homologous recombination crossover.

According to one embodiment of the present invention, the host cell may be a *Corynebacterium* sp. strain, for example, a *Corynebacterium glutamicum* DS1 strain.

Another aspect of the present invention provides a method for producing L-lysine, comprising steps of: a) culturing the *Corynebacterium glutamicum* mutant strain in a medium; and b) recovering L-lysine from the mutant strain or the medium in which the mutant strain has been cultured.

The culturing may be performed using a suitable medium and culture conditions known in the art, and any person skilled in the art may easily adjust and use the medium and the culture conditions. Specifically, the medium may be a liquid medium, without being limited thereto. The culturing method include may be, for example, batch culture, continuous culture, fed-batch culture, or a combination thereof, without being limited thereto.

According to one embodiment of the present invention, the medium should meet the requirements of a specific strain in a proper manner, and may be appropriately modified by a person skilled in the art. For the culture medium for a *Corynebacterium* sp. strain, reference may be made to a known document (Manual of Methods for General Bacteriology, American Society for Bacteriology, Washington D.C., USA, 1981), without being limited thereto.

According to one embodiment of the present invention, the medium may contain various carbon sources, nitrogen sources, and trace element components. Examples of the carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of the nitrogen sources that may be used include peptone, yeast extract, meat extract, malt extract, corn steep liquor, soybean meal, and urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be added to the culture medium. The medium or individual components may be added to the culture medium batchwise or in a continuous manner by a suitable method during culturing, without being limited thereto.

According to one embodiment of the present disclosure, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering L-lysine from the cultured mutant strain or the medium in which the mutant strain has been cultured, the produced L-lysine may be collected or recovered from the medium using a suitable method known in the art depending on the culture method. Examples of the method include, but are not limited to, centrifugation, filtration, extraction, spraying, drying, evaporation, precipitation, crystallization, electrophoresis, fractional dissolution (e.g., ammonium sulfate precipitation), and chromatography (e.g., ion exchange, affinity, hydrophobicity and size exclusion).

According to one embodiment of the present disclosure, the step of recovering L-lysine may be performed by centrifuging the culture medium at a low speed to remove biomass and separating the obtained supernatant through ion-exchange chromatography.

According to one embodiment of the present disclosure, the step of recovering L-lysine may include a process of purifying L-lysine.

### Advantageous Effects

The *Corynebacterium glutamicum* mutant strain according to the present invention is able to produce L-lysine in an improved yield as a result of increasing the supply of the L-lysine precursor and sugar utilization by increasing or enhancing the expression of the gene encoding enolase and/or reducing or weakening the expression of the gluconate operon transcriptional repressor.

### Brief Description of Drawings

FIG. 1 shows the structure of a pCG1+eno(G1A) vector containing an enolase (Eno) gene obtained by GTG-to-ATG replacement in the start codon according to one example of the present invention.
FIG. 2 shows the structure of a pCG1+gntR(S77F) vector containing a gntR gene obtained by substitution of phenylalanine for the amino acid serine at position 77 in the amino acid sequence of the gluconate operon transcriptional repressor (gntR) gene according to one example of the present invention.

### Mode for Invention

Hereinafter, the present invention will be described in more detail. However, this description is provided by way of example only to aid the understanding of the present invention, and the scope of the present invention is not limited by this illustrative description.

### Example 1. Construction of Corynebacterium glutamicum Mutant Strain

A *Corynebacterium glutamicum* DS1 strain and *E. coli* DH5a (HIT competent cells^{™}, Cat No. RH618) were used to construct a *Corynebacterium glutamicum* mutant strain having enhanced activity of enolase.

The *Corynebacterium glutamicum* DS1 strain was cultured in a CM-broth medium (pH 6.8) containing, per liter of distilled water, 5 g of glucose, 2.5 g of NaCl, 5.0 g of yeast extract, 1.0 g of urea, 10.0 g of polypeptone and 5.0 g of beef extract at a temperature of 30°C.

The *E. coli* DH5a was cultured in an LB medium containing, per liter of distilled water, 10.0 g of tryptone, 10.0 g of NaCl and 5.0 g of yeast extract at a temperature of 37°C.

The antibiotics kanamycin and streptomycine used were purchased from Sigma.

DNA sequencing was performed by Macrogen.

### 1-1. Construction of Recombinant Vector

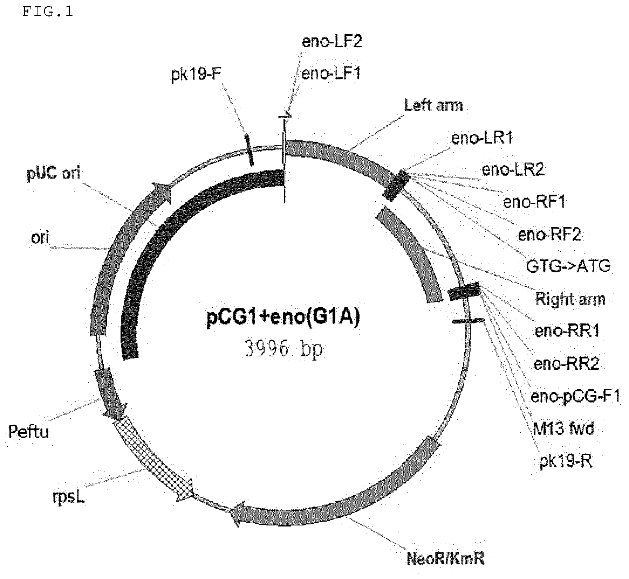

In order to increase the supply of the precursor to the TCA cycle in the strain, enhancement of enolase was introduced into the strain. In the method used in this Example, a specific mutation was induced in the translation start codon of the Eno gene encoding enolase in order to increase the expression of the Eno gene. The translation start codon of the Eno gene was mutated from GTG to ATG, and a 430-bp region of the left arm and a 439-bp region of the right arm with respect to the center of the Eno gene on the *Corynebacterium glutamicum* genome were amplified by PCR, ligated by overlap PCR, and then cloned into the recombinant vector pCGI (see Kim et al., Journal of Microbiological Methods 84 (2011) 128-130). The resulting plasmid was named pCG1+eno(G1A) (see FIG. 1). For construction of the plasmid, the primers shown in Table 1 below were used to amplify each gene fragment.

**[Table 1]**

| Primer (5'→3') | | | SEQ ID NO. |
|---|---|---|---|
| Primers for amplification of left homology arm of Eno | eno-LF1 | tgattacgccggcgacttatgggattggat | 9 |
| | eno-LF2 | ggcgacttatgggattggat | 10 |
| | eno-LR1 | tatgccaacttgggagctaa | 11 |
| | eno-LR2 | tgtggcctcctatgccaac | 12 |
| Primers for amplification of right homology arm of Eno | eno-RF1 | ggaggccacaatggctgaaatcatgcacgt | 13 |
| | eno-RF2 | atggctgaaatcatgcacgt | 14 |
| | eno-RR1 | aactggaagaacgtgtgcg | 15 |
| | eno-RR2 | tcatcattggaactggaagaac | 16 |

PCR was performed using the above primers under the following conditions. Using a thermocycler (TP600, TAKARA BIO Inc., Japan), and a reaction solution containing 100 µM of each deoxynucleotide triphosphate (dATP, dCTP, dGTP, dTTP), 1 pM of oligonucleotide, and 10 ng of the chromosomal DNA of *Corynebacterium glutamicum* ATCC 13032 as a template, PCR was performed for 25 to 30 cycles in the presence of 1 unit of a pfu-X DNA polymerase mixture (Solgent). The PCR cycles each consisted of (i) denaturation at 94°C for 30 sec, (ii) annealing at 58°C for 30 sec, and (iii) extension at 72°C for 1 to 2 min (a polymerization time of 2 min per kb).

The gene fragments produced as described above were cloned into the pCGI vector by self-assembly cloning. The vector was transformed into *E. coli* DH5a, which was then streaked on an LB-agar plate containing 50 µg/ml of kanamycin, and cultured at 37°C for 24 hours. After the finally formed colonies were isolated and it was checked whether the inserts would be exactly present in the vector, the vector was isolated and used for recombination of the *Corynebacterium glutamicum* strain.

As the process commonly performed in the above method, the genes of interest were amplified from the genomic DNA of *Corynebacterium glutamicum* ATCC 13032 by PCR and inserted into the pCGI vector by self-assembly cloning according to the strategy, followed by selection in *E*. *coli* DH5a. For chromosomal base substitution, the gene fragments were amplified individually and ligated by overlap PCR to obtain a target DNA fragment. During genetic manipulation, Ex Tag polymerase (Takara) and Pfu polymerase (Solgent) were used as PCR amplification enzymes, and various restriction enzymes and DNA modifying enzymes used were purchased from NEB. These polymerases and enzymes were used according to the supplied buffer and protocols.

### 1-2. Construction of Mutant Strain

A DS7-1 strain, a mutant strain, was constructed using the pCG1+eno(G1A) vector. The vector was prepared at a final concentration of 1 µg/µl or higher, and introduced into the *Corynebacterium glutamicum* DS1 strain by electroporation (see Tauch et al., FEMS Microbiology Letters 123 (1994), 343-347), thus inducing primary recombination. At this time, the electroporated strain was plated on a CM-agar plate containing 20 µg/µl of kanamycin, and the colonies were isolated, and then whether the vector would be properly inserted into the induced position on the genome was analyzed by PCR and sequencing. In order to induce secondary recombination, the isolated strain was inoculated into a CM-agar liquid medium containing streptomycin, cultured overnight or longer, and then plated on an agar medium containing streptomycin at the same concentration, and the colonies were isolated. After it was checked whether the final isolated colonies would have resistance to kanamycin, whether mutation was introduced into the Eno gene in the strains having no antibiotic resistance was analyzed by sequencing (see Schafer et al., Gene 145 (1994), 69-73). Finally, a *Corynebacterium glutamicum* mutant strain (DS7-1) having the mutant Eno gene introduced thereinto was obtained.

### Experimental Example 1. Comparison of L-Lysine Productivity Between Parent Strain and Mutant Strain

L-lysine productivity was compared between the parent strain *Corynebacterium glutamicum* DS1 strain and the L-lysine-producing mutant strain DS7-1 strain constructed in Examples 1.

The parent strain (DS1) or the mutant strain (DS7-1) was inoculated into a 100-ml flask containing 10 ml of a lysine medium having the composition shown in Table 2 below, and then cultured with shaking at 180 rpm at 30°C for 28 hours. After completion of the culture, the amount of L-lysine produced was measured by HPLC (Shimadzu, Japan), and the results of the measurement are shown in Table 3 below.

**[Table 2]**

| Composition | Content (per L of distilled water) |
|---|---|
| Glucose | 100 g |
| Ammonium sulfate | 55 g |
| KH₂PO₄ | 1.1 g |
| MgSO₄ · H₂O | 1.2 g |
| MnSO₄ · H₂O | 180 mg |
| FeSO₄ · H₂O | 180 mg |
| Thiamine · HCl | 9 mg |
| Biotin | 1.8 mg |
| CaCO₃ | 5% |
| pH | 7.0 |

**[Table 3]**

| Strain | L-lysine (g/L) | L-lysine production per gram dry cell weight (g/gDCW) |
|---|---|---|
| Parent strain (DS1) | 52.9 | 6.6 |
| Mutant strain (DS7-1) | 64.7 | 7.0 |

As shown in Table 3 above, it was confirmed that, in the *Corynebacterium glutamicum* mutant strain DS7-1 in which the start codon of the Eno gene was replaced with the optimal translation start sequence (ATG) to enhance the lysine biosynthesis pathway, the L-lysine productivity of the mutant strain increased by about 22% compared to that of the parent strain *Corynebacterium glutamicum* DS1 strain. From these results, it could be seen that enhanced expression of the Eno gene enhanced L-lysine productivity of the mutant strain by increasing the metabolic flux of carbon sources.

### Example 2. Construction of Corynebacterium glutamicum Mutant Strain

### 2-1. Construction of Recombinant Vector

In order to further increase the lysine productivity of the *Corynebacterium glutamicum* mutant strain constructed in Example 1, weakening of the gluconate operon transcriptional repressor that affects sugar utilization was introduced into the *Corynebacterium glutamicum* mutant strain DS7-1.

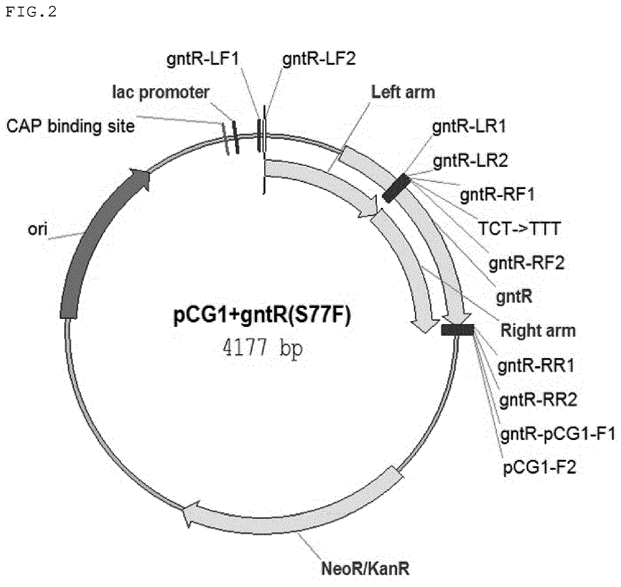

In the method used in this Example, a specific mutation in the gntR gene encoding the gluconate operon transcriptional repressor was induced in order to weaken the expression of the gntR gene. The amino acid serine at position 77 in the amino acid sequence of the gntR gene was substituted with phenylalanine, a 510-bp region of the left arm and a 540-bp region of the right arm with respect to the region including 77^{th} amino acid of the gntR gene on the *Corynebacterium glutamicum* genome were amplified by PCR, ligated by overlap PCR, and then cloned into the recombinant vector pCGI (see Kim et al., Journal of Microbiological Methods 84 (2011), 128-130). The resulting plasmid was named pCG1+gntR(S77F) (see FIG. 2). For construction of the plasmid, the primers shown in Table 4 below were used to amplify each gene fragment.

**[Table 4]**

| Primer (5'→3') | | | SEQ ID NO. |
|---|---|---|---|
| Primers for amplification of left homology arm of gntR | gntR-LF1 | tgattacgcccatcgaccgcctccgtat | 17 |
| | gntR-LF2 | catcgaccgcctccgtat | 18 |
| | gntR-LR1 | cgacaagaccgagctgct | 19 |
| | gntR-LR2 | cgtgaaaaagcgacaagacc | 20 |
| Primers for amplification of right homology arm of gntR | gntR-RF1 | ctttttcacgtcgcattggcattactgttt | 21 |
| | gntR-RF2 | tcgcattggcattactgttt | 22 |
| | gntR-RR1 | tgcgcgtatcacgttagttc | 23 |
| | gntR-RR2 | gcaaacgcagtgcgcgtat | 24 |

The processes of amplifying and cloning the genes were performed in the same manner as in Example 1-1.

### 2-2. Construction of Mutant Strain

Strain DS7-2, a mutant strain, was constructed using the pCG1+gntR(S77F) vector. The *Corynebacterium glutamicum* mutant strain (DS7-2) having a mutant gntR gene introduced thereinto was finally obtained by performing the same method as in Example 1-2, except that pCG1+gntR(S77F) was used instead of pCG1+eno(G1A) as a vector and the *Corynebacterium glutamicum* mutant strain DS7-1 was used instead of the *Corynebacterium glutamicum* DS1 strain as the host cell.

### Experimental Example 2. Comparison of L-Lysine Productivity between Parent Strain and Mutant Strain

L-lysine productivity was compared between the lysine-producing mutant strain DS7-1 strain constructed in Example 1 and the lysine-producing mutant strain DS7-2 strain constructed in Example 2 using the DS7-1 strain as the parent strain.

Measurement of L-lysine production was performed in the same manner as Experimental Example 1, and the results are shown in Table 5 below.

**[Table 5]**

| Strain | L-lysine (g/L) | L-lysine production per gram dry cell weight (g/gDCW) |
|---|---|---|
| Patent strain (DS7-1) | 63.8 | 6.9 |
| Mutant strain (DS7-2) | 65.3 | 7.4 |

**As** shown in Table 5 above, it was confirmed that, in the *Corynebacterium glutamicum* mutant strain DS7-2 in which the start codon of the Eno gene was replaced with the optimal translation start sequence (ATG) to enhance the lysine biosynthesis pathway and in which the amino acid at a specific position (77^{th} amino acid) in the amino acid sequence of the gntR gene was substituted with the optimal amino acid, the L-lysine productivity of the mutant strain increased by about 2% compared to that of the *Corynebacterium glutamicum* DS7-1 strain used as the parent strain. From these results, it could be seen that enhanced expression of the Eno gene and weakened expression of the gntR gene enhanced L-lysine productivity of the mutant strain by increasing the metabolic flux of carbon sources.

So far, the present invention has been described with reference to the embodiments thereof. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

## Claims

1. A *Corynebacterium glutamicum* mutant strain having enhanced L-lysine productivity by having enhanced activity of enolase.

2. The *Corynebacterium glutamicum* mutant strain of claim 1, wherein the enhanced activity of the enolase is achieved by site-directed mutagenesis of a gene encoding the enolase.

3. The *Corynebacterium glutamicum* mutant strain of claim 1, wherein the enhanced activity of the enolase is achieved by replacement of a start codon of a gene encoding the enolase with ATG.

4. The *Corynebacterium glutamicum* mutant strain of claim 3, wherein the mutant strain comprises a nucleotide sequence represented by SEQ ID NO: 3.

5. The *Corynebacterium glutamicum* mutant strain of claim 1, wherein the mutant strain further has weakened activity of gluconate operon transcriptional repressor.

6. The *Corynebacterium glutamicum* mutant strain of claim 5, wherein the weakened activity of the gluconate operon transcriptional repressor is achieved by site-directed mutagenesis of a gene encoding the gluconate operon transcriptional repressor.

7. The *Corynebacterium glutamicum* mutant strain of claim 6, wherein the mutant strain comprises an amino acid sequence represented by SEQ ID NO: 8.

8. A method for producing L-lysine, comprising steps of:
a) culturing the *Corynebacterium glutamicum* mutant strain of any one of claims 1 to 7 in a medium; and
b) recovering L-lysine from the mutant strain or the medium in which the mutant strain has been cultured.
